# EUROPEAN PATENT SPECIFICATION

(11) **EP 3 796 966 B1**
(45) Date of publication and mention of the grant of the patent: **28.08.2024**
(21) Application number: 19745865.6
(22) Date of filing: 05.07.2019
(51) Int. Cl.: A61N 1/05, A61N 1/36, A61N 1/375

(54) **DIRECTIONAL ELECTRICAL STIMULATION LEADS AND SYSTEMS FOR SPINAL CORD STIMULATION**
GERICHTETE ELEKTRISCHE STIMULATIONSLEITUNGEN UND SYSTEME ZUR RÜCKENMARKSTIMULATION
FILS DIRECTIONNELS DE STIMULATION ÉLECTRIQUE, ET SYSTÈMES POUR LA STIMULATION DE LA MOELLE ÉPINIÈRE

(30) Priority: 09.07.2018 US 201862695670 P
(43) Date of publication of application: 31.03.2021
(73) Proprietor: Boston Scientific Neuromodulation Corporation, Valencia, CA 91355 (US)
(72) Inventor: HOWARD, Joshua Dale, Sacramento, CA 95820 (US); BRILL, Natalie A., Sherman Oaks, CA 91403 (US); MOFFITT, Michael A., Solon, OH 44139 (US); LEVEN, Jacob B., Huntington Beach, CA 92647 (US)
(74) Representative: Vossius & Partner Patentanwälte Rechtsanwälte mbB
(86) International application number: PCT/US2019/040689
(87) International publication number: WO 2020/014083

(56) References cited:
- US-A1- 2010 057 176
- US-A1- 2013 289 683
- US-A1- 2015 025 609
- US-B2- 8 600 509

## Description

### FIELD

The present disclosure is directed to the area of implantable electrical stimulation systems and methods of making and using the systems. The present disclosure is also directed to directional electrical stimulation leads, systems, and methods for spinal cord stimulation.

### BACKGROUND

Implantable electrical stimulation systems have proven therapeutic in a variety of diseases and disorders. For example, spinal cord stimulation systems have been used as a therapeutic modality for the treatment of chronic pain syndromes. Sacral nerve stimulation has been used to treat incontinence, as well as a number of other applications under investigation. Functional electrical stimulation systems have been applied to restore some functionality to paralyzed extremities in spinal cord injury patients.

Stimulators have been developed to provide therapy for a variety of treatments. A stimulator can include a control module (with a pulse generator), one or more leads, and an array of stimulator electrodes on each lead. The stimulator electrodes are in contact with or near the nerves, muscles, or other tissue to be stimulated. The pulse generator in the control module generates electrical pulses that are delivered by the electrodes to body tissue.

US 2010/0057176 A1 discloses an insertion kit that includes a lead and a splitable member configured and arranged for receiving the lead when implanting the lead into a patient. The lead has a distal end and at least two proximal ends. The lead includes a plurality of electrodes disposed at the distal end, a plurality of terminals disposed at the proximal ends, and a plurality of conductive wires coupling the plurality of electrodes electrically to the plurality of terminals. The lead also includes a junction coupling the distal end of the lead to the proximal ends of the lead. The splitable member defines a lumen for receiving the distal end of the lead and is configured and arranged to divide into at least two parts for removal of the splitable member from the lead upon implantation of the lead into the patient.

### BRIEF SUMMARY

The invention is defined in the appended claims. Aspects, embodiments and examples disclosed herein which do not fall within the scope of the appended claims do not form part of the invention, and are merely provided for illustrative purposes.

The present invention relates to a lead arrangement that includes an electrical stimulation lead having a proximal portion, a distal portion, and a medial portion between the proximal portion and the distal portion. The electrical stimulation lead includes electrodes disposed along the distal portion of the electrical stimulation lead, proximal terminals disposed along the proximal portion of the electrical stimulation lead, medial terminals disposed along the medial portion of the electrical stimulation lead, first conductors extending along the electrical stimulation lead and electrically coupling some of the electrodes to the proximal terminals, and second conductors extending along the electrical stimulation lead and electrically coupling some of the electrodes to the medial terminals, wherein an outer diameter of a first portion of the electrical stimulation lead distal to the medial terminals and proximal to the electrodes is larger than an outer diameter of a second portion of the electrical stimulation lead proximal to the medial terminals. The lead arrangement also includes an extension having a proximal portion and a distal portion. The extension includes extension terminals disposed along the proximal portion of the extension, and an extension connector disposed along the distal portion of the extension. The extension connector includes a connector housing defining a central lumen configured to receive the medial portion of the electrical stimulation lead, and connector contacts disposed within the connector housing and along the central lumen, wherein an inner diameter of a first portion of the central lumen distal to the connector contacts is larger than an inner diameter of a second portion of the central lumen proximal to the connector contacts to limit insertion of the electrical stimulation lead through the extension connector.

In at least some aspects, the extension connector further includes a distal entrance element disposed within the housing and defining a distal-most portion of the central lumen having the larger inner diameter. In at least some aspects, the extension connector further includes a proximal entrance element disposed within the housing and defining a proximal-most portion of the central lumen. In at least some aspects, the extension connector further includes a plurality of spacers, each spacer disposed between adjacent ones of the connector contacts. In at least some aspects, the electrical stimulation lead includes at least twenty electrodes.

In at least some aspects, the electrical stimulation lead includes a multi-lumen conductor guide disposed at least between the proximal terminals and the medial terminals, the multi-lumen conductor guide defining conductor lumens disposed around the central lumen with each of the conductor lumens including a portion of one or more of the second conductors disposed therein. In at least some aspects, the first conductors and at least a portion of the second conductors are disposed in a single layer, coiled arrangement between at least the medial terminals and the electrodes. In at least some aspects, the first conductors and at least a portion of the second conductors are disposed in a two layer, coiled arrangement between at least the medial terminals and the electrodes. In at least some aspects, the two layer, coiled arrangement includes a first layer and a second layer, wherein the first conductors are coiled in the first layer and the second conductors are coiled in the second layer.

The present invention further relates to a system for electrical stimulation that includes any of the lead arrangements described above and a control module electrically coupleable to the electrical stimulation lead and the extension.

Further disclosed is an electrical stimulation lead that includes a lead body having a proximal portion, a straight distal portion, and a bent distal portion; first electrodes disposed along the straight distal portion of the lead body; second electrodes disposed along the bent distal portion of the lead body; terminals disposed along the proximal portion of the lead body; and conductors extending along the electrical stimulation lead and electrically coupling some of the first and second electrodes to the terminals.

In at least some aspects, the electrical stimulation lead is configured for insertion into the epidural space of the spinal cord with the bent distal portion passing through a foramen and into position near a dorsal root or dorsal root ganglion. In at least some aspects, the electrical stimulation lead is configured for insertion into the epidural space of the spinal cord with the straight distal portion disposed along a midline of the spinal cord and the bent distal portion positioning at least one electrode over a dorsal horn, rootlet, or root of the spinal cord. In at least some aspects, the lead body further includes a second straight distal portion distal to the bent distal portion with the at least one electrode disposed on the second straight distal portion. In at least some aspects, the first electrodes and the plurality of second electrodes include, in total, at least twenty electrodes and wherein, optionally, the conductors are disposed in a two layer, coiled arrangement between at least the terminals and the first and second electrodes.

Further disclosed is a system for electrical stimulation that includes any of the electrical stimulation leads described above and a control module electrically coupleable to the electrical stimulation lead.

Further disclosed is a method of stimulating a spinal cord of a patient. The method includes providing an electrical stimulation lead implanted within an epidural space of the patient. The electrical stimulation lead includes a lead body having a proximal portion and a distal portion and a circumference, electrodes disposed along the distal portion of the lead body; terminals disposed along the proximal portion of the lead body; and conductors extending along the electrical stimulation lead and electrically coupling the electrodes to the terminals, the electrodes including at least one set of segmented electrodes disposed at longitudinal position on the lead body with each of the segmented electrodes extending around less than half of the circumference of the lead body. The method also includes producing medial stimulation of the spinal cord using one or more of the plurality of electrodes at a first longitudinal position along the lead body and producing only one of left lateral stimulation or right lateral stimulation of the spinal cord using one or more of the plurality of electrodes at a second longitudinal position along the lead body.

In at least some aspects, the medial stimulation and the left or right lateral stimulation are produced simultaneously at different longitudinal positions along the lead body.

In at least some aspects, the distal portion of the lead body includes a straight distal portion and a bent distal portion that is distal to the straight distal portion with at least one or more electrodes disposed along each of the straight distal portion and the bent distal portion, wherein the electrical stimulation lead is implanted with the straight distal portion disposed over a midline of the spinal cord and the bent distal portion positions at least one of the electrodes over a dorsal horn, rootlet, or root of the spinal cord, the method further including: producing dorsal column stimulation of the spinal cord using one or more of the plurality of electrodes along the straight distal portion of the lead body; and producing dorsal horn, rootlet, or root stimulation of the spinal cord using one or more of the at least one of the electrodes positioned over the dorsal horn, rootlet, or root of the spinal cord.

In at least some aspects, the distal portion of the lead body includes a straight distal portion and a bent distal portion that is distal to the straight distal portion with at least one or more electrodes disposed along each of the straight distal portion and the bent distal portion, wherein the electrical stimulation lead is implanted with the straight distal portion disposed within the epidural space of the spinal cord and the bent distal portion extending through a foramen to position at least one of the electrodes over a dorsal root or dorsal root ganglion, the method further including: producing spinal cord stimulation using one or more of the plurality of electrodes along the straight distal portion of the lead body; and producing dorsal root or dorsal root ganglion stimulation of the spinal cord using one or more of the at least one of the electrodes positioned over the dorsal root or dorsal root ganglion.

### BRIEF DESCRIPTION OF THE DRAWINGS

Non-limiting and non-exhaustive embodiments of the present invention are described with reference to the following drawings. In the drawings, like reference numerals refer to like parts throughout the various figures unless otherwise specified.

For a better understanding of the present invention, reference will be made to the following Detailed Description, which is to be read in association with the accompanying drawings, wherein:
FIG. 1 is a schematic view of another embodiment of an electrical stimulation system that includes a percutaneous lead body coupled to a control module;
FIG. 2A is a schematic view of one embodiment of a plurality of connector assemblies disposed in the control module of FIG. 1A, the connector assemblies configured and arranged to receive the proximal portions of the lead bodies of FIG. 1A;
FIG. 2B is a schematic view of one embodiment of a proximal portion of the lead body of FIG. 1, a lead extension, and the control module of FIG. 1A, the lead extension configured and arranged to couple the lead body to the control module;
FIG. 3A is a schematic perspective view of a portion of one embodiment of a lead with thirty-two electrodes;
FIG. 3B is a schematic perspective view of portions of one embodiment of two leads each with sixteen electrodes;
FIG. 3C is a schematic perspective view of portions of another embodiment of two leads each with sixteen electrodes;
FIG. 3D is a schematic perspective view of portions of a third embodiment of two leads each with sixteen electrodes;
FIG. 3E is a schematic perspective view of a portion of another embodiment of a lead with thirty-two electrodes;
FIG. 3F is a schematic perspective view of a portion of one embodiment of a lead with electrodes and a tip stimulator;
FIG. 4A is a schematic cross-section of one embodiment of a lead with coiled conductors;
FIG. 4B is a schematic cross-section of another embodiment of a lead with coiled conductors;
FIG. 4C is a schematic cross-section of one embodiment of a lead with a multi-lumen conductor guide;
FIG. 5A is a schematic transverse cross-sectional view of spinal nerves extending from a spinal cord, the spinal nerves including dorsal root and dorsal root ganglia;
FIG. 5B is a schematic perspective view of a portion of the spinal cord of FIG. 5A disposed in a portion of a vertebral column with the dorsal root and dorsal root ganglia of FIG. 5A extending outward from the vertebral column;
FIG. 5C is a schematic top view of a portion of the spinal cord of FIG. 5A disposed in a vertebral foramen defined in a vertebra of the vertebral column of FIG. 5B, the vertebra also defining intervertebral foramina extending between an outer surface of the vertebra and the vertebral foramen, the intervertebral foramina providing an opening through which the dorsal root and dorsal root ganglia of FIG. 5B can extend outward from the spinal cord of FIG. 5B,
FIG. 5D is a schematic view of one embodiment of a lead with segmented electrodes inserted through the epidural space of the spinal cord of FIG. 5A;
FIG. 5E is a schematic view of one embodiment of two leads with segmented electrodes inserted through the epidural space of the spinal cord of FIG. 5A;
FIG. 5F is a schematic view of one embodiment of a lead with a straight distal section and a bent distal section inserted through the epidural space of the spinal cord of FIG. 5A;
FIG. 5G is a schematic view of one embodiment of a lead with a straight distal section and a bent distal section inserted through the epidural space of the spinal cord of FIG. 5A with the bent distal section extending through a foramen to a dorsal root or dorsal root ganglion;
FIG. 6A is a schematic perspective view of one embodiment of a lead arrangement including an electrical stimulation lead and an extension with a medial connector;
FIG. 6B is a schematic perspective view of the electrical stimulation lead of the lead arrangement of FIG. 6A;
FIG. 6C is a schematic perspective view of the extension of the lead arrangement of FIG. 6A;
FIG. 6D is a schematic perspective view of the medial connector of the lead arrangement of FIG. 6A;
FIG. 7A is a schematic perspective view of another embodiment of a lead arrangement including an electrical stimulation lead and an extension with a medial connector;
FIG. 7B is a schematic perspective view of the extension of the lead arrangement of FIG. 7A; and
FIG. 8 is a schematic overview of one embodiment of components of an electrical stimulation system.

### DETAILED DESCRIPTION

The present disclosure is directed to the area of implantable electrical stimulation systems and methods of making and using the systems. The present disclosure is also directed to directional electrical stimulation leads, systems, and methods for spinal cord stimulation.

Suitable implantable electrical stimulation systems include, but are not limited to, a least one lead with one or more electrodes disposed along a distal end of the lead and one or more terminals disposed along the one or more proximal ends of the lead.

Examples of electrical stimulation systems with leads are found in, for example, U.S. Patents Nos. 6,181,969; 6,295,944; 6,391,985; 6,516,227; 6,609,029; 6,609,032; 6,741,892; 7,244,150; 7,450,997; 7,672,734;7,761,165; 7,783,359; 7,792,590; 7,809,446; 7,949,395; 7,974,706; 8,831,742; 8,688,235; 6,175,710; 6,224,450; 6,271,094; 6,295,944; 6,364,278; and 6,391,985; U.S. Patent Application Publications Nos. 2007/0150036; 2009/0187222; 2009/0276021; 2010/0076535; 2010/0268298; 2011/0004267; 2011/0078900; 2011/0130817; 2011/0130818; 2011/0238129; 2011/0313500; 2012/0016378; 2012/0046710; 2012/0071949; 2012/0165911; 2012/0197375; 2012/0203316; 2012/0203320; 2012/0203321; 2012/0316615; 2013/0105071; 2011/0005069; 2010/0268298; 2011/0130817; 2011/0130818; 2011/0078900; 2011/0238129; 2011/0313500; 2012/0016378; 2012/0046710; 2012/0165911; 2012/0197375; 2012/0203316; 2012/0203320; and 2012/020332 1.

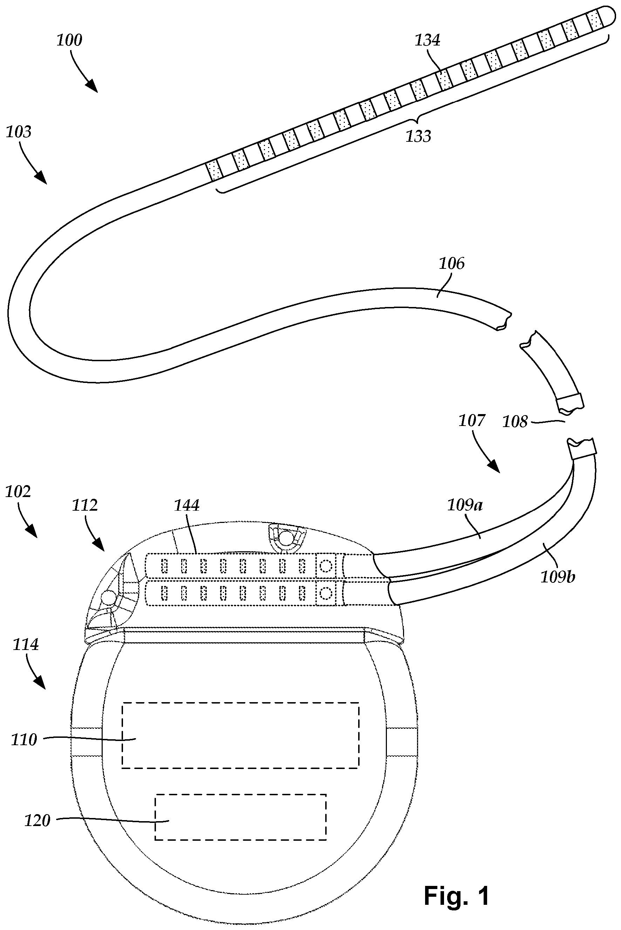

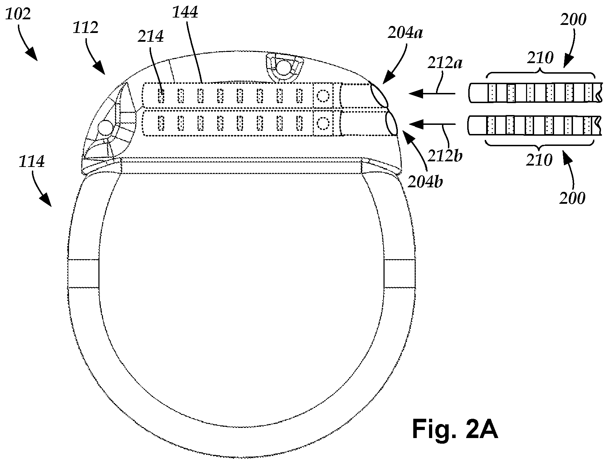

Figure 1 illustrates schematically one embodiment of an electrical stimulation system 100. The electrical stimulation system includes a control module (e.g., a stimulator or pulse generator) 102 and at least one lead 103 coupleable to the control module 102. The lead 103 includes one or more lead bodies 106, an array of electrodes 133, such as electrode 134, and an array of terminals (*e*.*g*., 210 in Figure 2A-2B) disposed along the one or more lead bodies 106. In at least some embodiments, the lead is isodiametric along a longitudinal length of the lead body 106. Figure 1 illustrates one lead 103 coupled to a control module 102. Other embodiments may include two, three, four, or more leads 103 coupled to the control module 102.

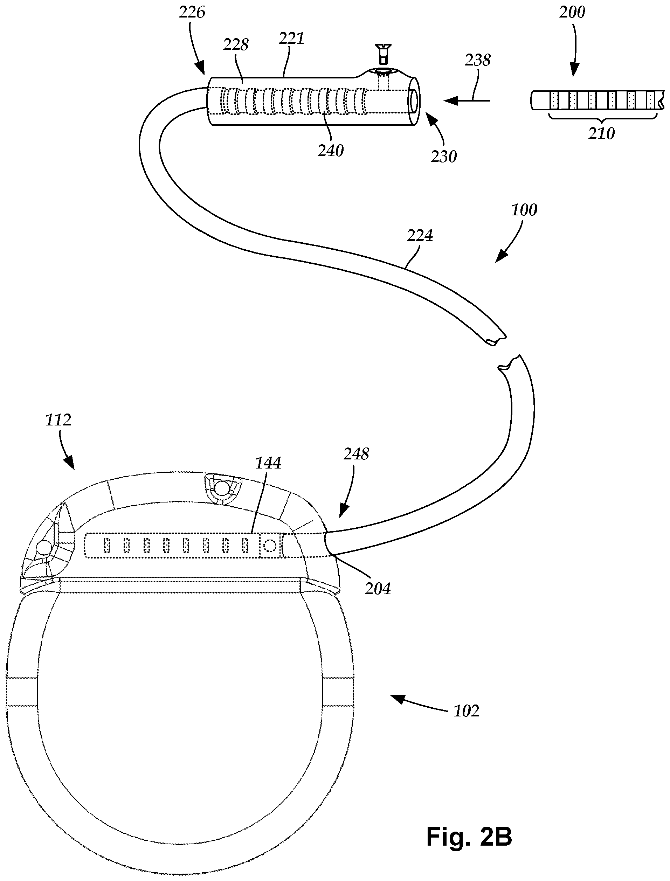

The lead 103 can be coupled to the control module 102 in any suitable manner. In at least some embodiments, the lead 103 couples directly to the control module 102. In at least some other embodiments, the lead 103 couples to the control module 102 via one or more intermediate devices. For example, in at least some embodiments one or more lead extensions 224 (*see e.g.,* Figure 2B) can be disposed between the lead 103 and the control module 102 to extend the distance between the lead 103 and the control module 102. Other intermediate devices may be used in addition to, or in lieu of, one or more lead extensions including, for example, a splitter, an adaptor, or the like or combinations thereof. It will be understood that, in the case where the electrical stimulation system 100 includes multiple elongated devices disposed between the lead 103 and the control module 102, the intermediate devices may be configured into any suitable arrangement.

In Figure 1, the electrical stimulation system 100 is shown having a splitter 107 configured and arranged for facilitating coupling of the lead 103 to the control module 102. The splitter 107 includes a splitter connector 108 configured to couple to a proximal end of the lead 103, and one or more splitter tails 109a and 109b configured and arranged to couple to the control module 102 (or another splitter, a lead extension, an adaptor, or the like).

The control module 102 typically includes a connector housing 112 and a sealed electronics housing 114. An electronic subassembly 110 and an optional power source 120 are disposed in the electronics housing 114. A control module connector 144 is disposed in the connector housing 112. The control module connector 144 is configured and arranged to make an electrical connection between the lead 103 and the electronic subassembly 110 of the control module 102.

The electrical stimulation system or components of the electrical stimulation system, including one or more of the lead bodies 106 and the control module 102, are typically implanted into the body of a patient. The electrical stimulation system can be used for a variety of applications including, but not limited to, brain stimulation, neural stimulation, spinal cord stimulation, muscle stimulation, and the like.

The electrodes 134 can be formed using any conductive, biocompatible material. Examples of suitable materials include metals, alloys, conductive polymers, conductive carbon, and the like, as well as combinations thereof. In at least some embodiments, one or more of the electrodes 134 are formed from one or more of: platinum, platinum iridium, palladium, palladium rhodium, or titanium. The number of electrodes 134 in each array 133 may vary. For example, there can be two, four, six, eight, ten, twelve, fourteen, sixteen, or more electrodes 134. As will be recognized, other numbers of electrodes 134 may also be used.

The electrodes of the one or more lead bodies 106 are typically disposed in, or separated by, a non-conductive, biocompatible material such as, for example, silicone, polyurethane, polyetheretherketone ("PEEK"), epoxy, and the like or combinations thereof. The lead bodies 106 may be formed in the desired shape by any process including, for example, molding (including injection molding), casting, and the like. The non-conductive material typically extends from the distal end of the one or more lead bodies 106 to the proximal end of each of the one or more lead bodies 106.

Terminals (e.g., 210 in Figures 2A-2B) are typically disposed along the proximal end of the one or more lead bodies 106 of the electrical stimulation system 100 (as well as any splitters, lead extensions, adaptors, or the like) for electrical connection to corresponding connector contacts (e.g., 214 in Figure 2A and 240 in Figure 2B). The connector contacts are disposed in connectors (e.g., 144 in Figures 1-2B; and 221 in Figure 2B) which, in turn, are disposed on, for example, the control module 102 (or a lead extension, a splitter, an adaptor, or the like). Electrically conductive wires, cables, or the like (not shown) extend from the terminals to the electrodes 134. Typically, one or more electrodes 134 are electrically coupled to each terminal. In at least some embodiments, each terminal is only connected to one electrode 134.

The electrically conductive wires ("conductors") may be embedded in the non-conductive material of the lead body 106 or can be disposed in one or more lumens (not shown) extending along the lead body 106. In some embodiments, there is an individual lumen for each conductor. In other embodiments, two or more conductors extend through a lumen. There may also be one or more lumens (not shown) that open at, or near, the proximal end of the lead body 106, for example, for inserting a stylet to facilitate placement of the lead body 106 within a body of a patient. Additionally, there may be one or more lumens (not shown) that open at, or near, the distal end of the lead body 106, for example, for infusion of drugs or medication into the site of implantation of the one or more lead bodies 106. In at least one embodiment, the one or more lumens are flushed continually, or on a regular basis, with saline, epidural fluid, or the like. In at least some embodiments, the one or more lumens are permanently or removably sealable at the distal end.

Figure 2A is a schematic side view of one embodiment of a proximal end of one or more elongated devices 200 configured and arranged for coupling to one embodiment of the control module connector 144. The one or more elongated devices may include, for example, the lead body 106, one or more intermediate devices (*e*.*g*., the splitter 107 of Figure 1, the lead extension 224 of Figure 2B, an adaptor, or the like or combinations thereof), or a combination thereof. Figure 2A illustrates two elongated devices 200 coupled to the control module 102. These two elongated devices 200 can be two tails as illustrated in Figure 1 or two different leads or any other combination of elongated devices.

The control module connector 144 defines at least one port into which a proximal end of the elongated device 200 can be inserted, as shown by directional arrows 212a and 212b. In Figure 2A (and in other figures), the connector housing 112 is shown having two ports 204a and 204b. The connector housing 112 can define any suitable number of ports including, for example, one, two, three, four, five, six, seven, eight, or more ports.

The control module connector 144 also includes a plurality of connector contacts, such as connector contact 214, disposed within each port 204a and 204b. When the elongated device 200 is inserted into the ports 204a and 204b, the connector contacts 214 can be aligned with a plurality of terminals 210 disposed along the proximal end(s) of the elongated device(s) 200 to electrically couple the control module 102 to the electrodes (134 of Figure 1) disposed at a distal end of the lead 103. Examples of connectors in control modules are found in, for example, U.S. Patent No. 7,244,150 and 8,224,450.

Figure 2B is a schematic side view of another embodiment of the electrical stimulation system 100. The electrical stimulation system 100 includes a lead extension 224 that is configured and arranged to couple one or more elongated devices 200 (*e.g.,* the lead body 106, the splitter 107, an adaptor, another lead extension, or the like or combinations thereof) to the control module 102. In Figure 2B, the lead extension 224 is shown coupled to a single port 204 defined in the control module connector 144. Additionally, the lead extension 224 is shown configured and arranged to couple to a single elongated device 200. In alternate embodiments, the lead extension 224 is configured and arranged to couple to multiple ports 204 defined in the control module connector 144, or to receive multiple elongated devices 200, or both.

A lead extension connector 221 is disposed on the lead extension 224. In Figure 2B, the lead extension connector 221 is shown disposed at a distal end 226 of the lead extension 224. The lead extension connector 221 includes a connector housing 228. The connector housing 228 defines at least one port 230 into which terminals 210 of the elongated device 200 can be inserted, as shown by directional arrow 238. The connector housing 228 also includes a plurality of connector contacts, such as connector contact 240. When the elongated device 200 is inserted into the port 230, the connector contacts 240 disposed in the connector housing 228 can be aligned with the terminals 210 of the elongated device 200 to electrically couple the lead extension 224 to the electrodes (134 of Figure 1) disposed along the lead (103 in Figure 1).

In at least some embodiments, the proximal end of the lead extension 224 is similarly configured and arranged as a proximal end of the lead 103 (or other elongated device 200). The lead extension 224 may include a plurality of electrically conductive wires (not shown) that electrically couple the connector contacts 240 to a proximal end 248 of the lead extension 224 that is opposite to the distal end 226. In at least some embodiments, the conductive wires disposed in the lead extension 224 can be electrically coupled to a plurality of terminals (not shown) disposed along the proximal end 248 of the lead extension 224. In at least some embodiments, the proximal end 248 of the lead extension 224 is configured and arranged for insertion into a connector disposed in another lead extension (or another intermediate device). In other embodiments (and as shown in Figure 2B), the proximal end 248 of the lead extension 224 is configured and arranged for insertion into the control module connector 144.

Returning to Figure 1, the illustrated lead includes sixteen ring electrodes 134. Any number of ring electrodes can be disposed along the length of the lead body including, for example, one, two three, four, five, six, seven, eight, nine, ten, eleven, twelve, thirteen, fourteen, fifteen, sixteen or more ring electrodes. It will be understood that any number of ring electrodes can be disposed along the length of the lead body.

Conventional commercial spinal cord stimulation leads are either paddle leads, which typically require more invasive surgical methods to implant, or percutaneous leads with eight or sixteen ring electrodes.

In spinal cord stimulation, it may be useful to have an array 133 of electrodes 134 that spans multiple vertebral levels. In one embodiment, a lead 103 includes 32 electrodes 134 forming an array 134 and may span three, four, five or more vertebral levels. The electrodes 133 may have any suitable longitudinal length including, but not limited to, 2, 3, 4, 4.5, 5, or 6 mm. The longitudinal spacing between electrodes 133 may also be any suitable amount including, but not limited to, 1, 2, or 3 mm, where the spacing is defined as the distance between the nearest edges of two adjacent electrodes.

In some embodiments, the spacing is uniform between adjacent pairs of electrodes along the length of the lead. In other embodiments, because different vertebral levels have different lengths, the spacing between adjacent electrodes may be different or non-uniform along the length of the lead. For example, electrodes that will be positioned nearer the head or neck (for example, the cervical vertebrae) may have a smaller spacing (for example, 1 mm) between electrodes than the spacing (for example, 2, 2.25, or 2.5 mm) between those electrodes positioned near the lower thoracic or lumbar vertebrae. Furthermore, The cerebral spinal fluid (CSF) thickness can vary as a function of vertebral level, and therefore the electrode spacing can be different at different vertebral levels. Tighter electrode spacing is may be preferred for thinner CSF thickness.

Ring electrodes send current into all of the epidural space surrounding the electrode often including regions that are not the target of stimulation. In addition to, or as an alternative to, ring electrodes, a lead may include one or more segmented electrodes which extend only part of the way around the circumference of the lead (for example, less than one half or one third of the circumference of the lead. Segmented electrodes may provide for superior current steering than ring electrodes because target structures may not be disposed symmetrically about the axis of the distal electrode array. Instead, a target may be located on one side of a plane running through the axis of the lead. Through the use of a radially segmented electrode array ("RSEA"), current steering can be performed not only along a length of the lead but also around a circumference of the lead. This provides precise three-dimensional targeting and delivery of the current stimulus to target tissue, while potentially avoiding stimulation of other tissue.

Examples of leads with segmented electrodes include U.S. Patent Application Publications Nos. 2010/0268298; 2011/0005069; 2011/0078900; 2011/0130803; 2011/0130816; 2011/0130817; 2011/0130818; 2011/0078900; 2011/0238129; 2011/0313500; 2012/0016378; 2012/0046710; 2012/0071949; 2012/0165911; 2012/197375; 2012/0203316; 2012/0203320; 2012/0203321; 2013/0197602; 2013/0261684; 2013/0325091; 2013/0317587; 2014/0039587; 2014/0353001; 2014/0358209; 2014/0358210; 2015/0018915; 2015/0021817; 2015/0045864; 2015/0021817; 2015/0066120; 2013/0197424; 2015/0151113; 2014/0358207; and U.S. Patent No. 8,483,22 7.

A lead may also include a tip electrode and examples of leads with tip electrodes include at least some of the previously cited references, as well as U.S. Patent Application Publications Nos. 2014/0296953 and 2014/0343647, all of which are incorporated herein by reference in their entireties. A lead with segmented electrodes may be a directional lead that can provide stimulation in a particular direction using the segmented electrodes.

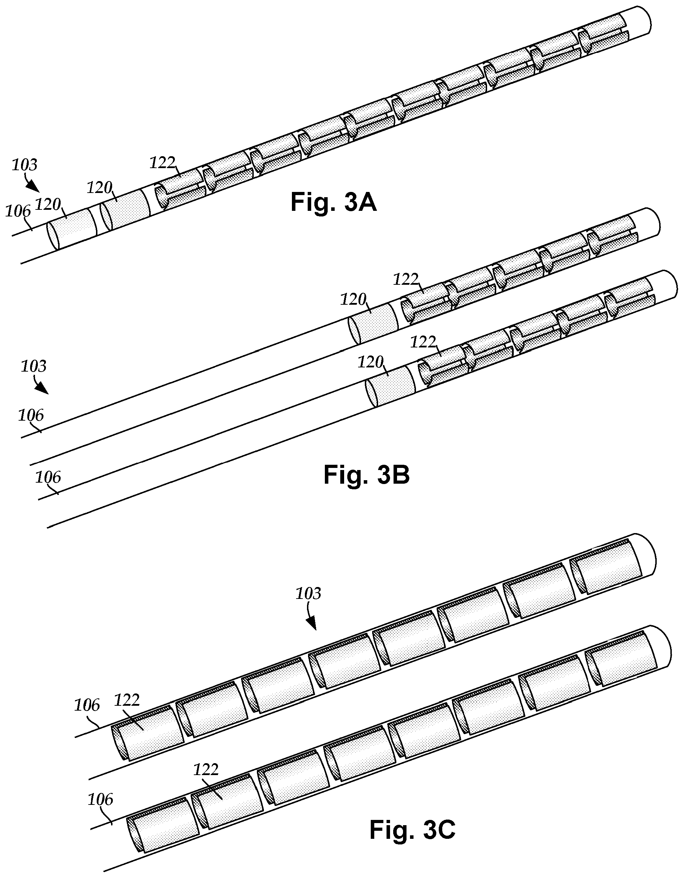

Figure 3A illustrates a 32-electrode lead 103 with a lead body 111 and two ring electrodes 120 proximal to thirty segmented electrodes 122 arranged in ten sets of three segmented electrodes each. In the illustrated embodiments, the ring electrodes 120 are proximal to the segmented electrodes 122. In other embodiments, the ring electrodes 120 can be proximal to, distal to, or between the segmented electrodes 122 or, when there is more than one ring electrode, each ring electrode can be positioned proximal to, distal to, or between the segmented electrodes.

Any number of segmented electrodes 122 may be disposed on the lead body including, for example, one, two, three, four, five, six, seven, eight, nine, ten, eleven, twelve, thirteen, fourteen, fifteen, sixteen, twenty, twenty-four, twenty-eight, thirty, thirty-two, or more segmented electrodes 122. It will be understood that any number of segmented electrodes 122 may be disposed along the length of the lead body. A segmented electrode 122 typically extends only 75%, 67%, 60%, 50%, 40%, 33%, 25%, 20%, 17%, 15%, or less around the circumference of the lead.

The segmented electrodes 122 may be grouped into sets of segmented electrodes, where each set is disposed around a circumference of the lead 103 at a particular longitudinal portion of the lead 103. The lead 103 may have any number of segmented electrodes 122 in a given set of segmented electrodes. The lead 103 may have one, two, three, four, five, six, seven, eight, or more segmented electrodes 122 in a given set. The lead 103 may have any number of sets of segmented electrode including, but not limited to, one, two, three, four, five, six, eight, ten, twelve, fifteen, sixteen, twenty, or more sets. The segmented electrodes 122 may be uniform, or vary, in size and shape. In some embodiments, the segmented electrodes 122 are all of the same size, shape, diameter, width or area or any combination thereof. In some embodiments, the segmented electrodes 122 of each circumferential set (or even all segmented electrodes disposed on the lead 103) may be identical in size and shape.

Each set of segmented electrodes 122 may be disposed around the circumference of the lead body to form a substantially cylindrical shape around the lead body. The spacing between individual electrodes of a given set of the segmented electrodes may be the same, or different from, the spacing between individual electrodes of another set of segmented electrodes on the lead 103. In at least some embodiments, equal spaces, gaps or cutouts are disposed between each segmented electrode 122 around the circumference of the lead body. In other embodiments, the spaces, gaps or cutouts between the segmented electrodes 122 may differ in size or shape. In other embodiments, the spaces, gaps, or cutouts between segmented electrodes 122 may be uniform for a particular set of the segmented electrodes 122, or for all sets of the segmented electrodes 122. The sets of segmented electrodes 122 may be positioned in irregular or regular intervals along a length of the lead body.

The electrodes of the lead 103 are typically disposed in, or separated by, a non-conductive, biocompatible material of a lead body 106 including, for example, silicone, polyurethane, and the like or combinations thereof. The lead body 106 may be formed in the desired shape by any process including, for example, extruding, molding (including injection molding), casting, and the like. Electrodes and connecting wires can be disposed onto or within a lead body either prior to or subsequent to a molding or casting process. The non-conductive material typically extends from the distal end of the lead body 106 to the proximal end of the lead body 106.

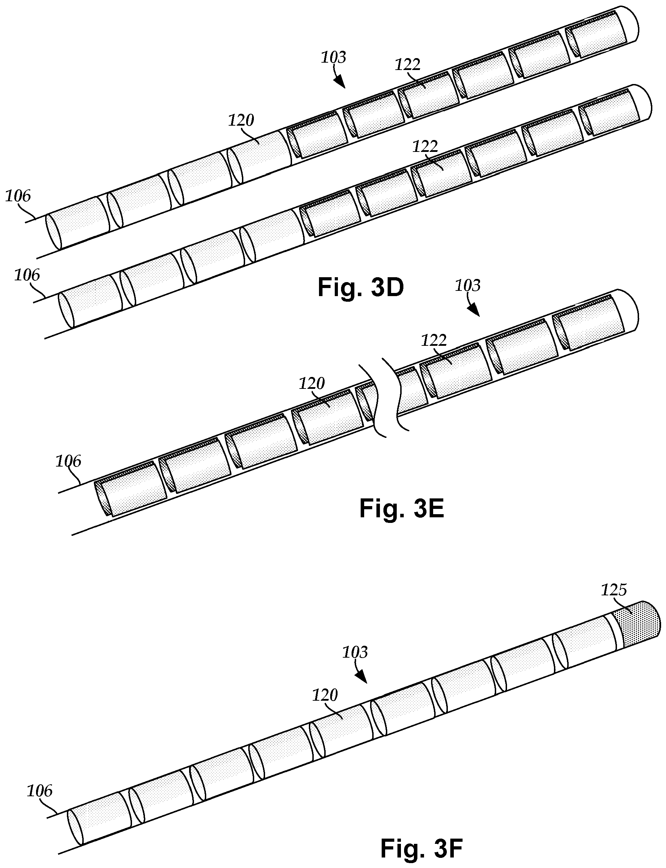

Figure 3B to 3E illustrate other embodiments of leads with segmented electrodes 122. Figure 3B illustrates two sixteen electrode leads 103 with each lead having one ring electrode 120 that is proximal to five sets of three segmented electrodes 122 each. Figure 3C illustrates two sixteen electrode leads 103 with each lead having eight sets of two segmented electrodes 122 each. As illustrated in Figure 3C, an embodiment of a lead 103 does not necessarily include a ring electrode. Figure 3D illustrates two sixteen electrode leads 103 with each lead having four ring electrodes 120 that are proximal to six sets of two segmented electrodes 122 each. Figure 3E illustrates a thirty-two electrode lead 103 having sixteen sets of two segmented electrodes 122 each (for clarity of illustration, not all of the electrodes are shown). It will be recognized that any other electrode combination of ring electrodes, segmented electrodes, or both types of electrodes can be used.

Figure 3F illustrates a lead 103 with a tip stimulator 125. In some embodiments, the tip stimulator 125 is a tip electrode. In other embodiments, the tip stimulator 125 can be an optical stimulator, such as a LED, OLED, laser diode, or other light emitter or a tip of an optical fiber or other optical waveguide from which light can be emitted. In addition to the tip stimulator 125, the lead 103 may also include ring electrodes, segmented electrodes, or both types of electrodes. For example, the lead 103 can include six, eight, ten, twelve, fourteen, sixteen, eighteen, twenty, twenty-two, twenty-four, or thirty electrodes and an optical tip stimulator. As other examples, the lead can include a tip stimulator and either a) thirty ring electrodes, b) twelve sets of two segmented electrodes each and six ring electrodes, or c) six sets of two segmented electrodes each and two ring electrodes.

The electrodes 120, 122 may have any suitable longitudinal length including, but not limited to, 2, 3, 4, 4.5, 5, or 6 mm. The longitudinal spacing between adjacent electrodes 120, 122 (as well as between an adjacent electrode 120, 122 and tip stimulator 125) may be any suitable amount including, but not limited to, 1, 2, or 3 mm, where the spacing is defined as the distance between the nearest edges of two adjacent electrodes. In some embodiments, the spacing is uniform between longitudinally adjacent of electrodes along the length of the lead. In other embodiments, because different vertebral levels have different lengths or different CSF thickness, the spacing between longitudinally adjacent electrodes may be different or non-uniform along the length of the lead. For example, electrodes that will be positioned nearer the head or neck (for example, the cervical vertebrae) may have a smaller longitudinal spacing (for example, 1 mm) between electrodes than the spacing (for example, 2, 2.25, or 2.5 mm) between those electrodes positioned near the lower thoracic or lumbar vertebrae.

In at least some commercial eight-electrode leads, the conductors extending from the terminals to the electrodes are arranged parallel to each other with each conductor (or a pair of conductors) disposed separate lumens of a multi-lumen conductor guide. Such an arrangement may be difficult for leads having more electrodes, where each electrode is attached to a separate conductor.

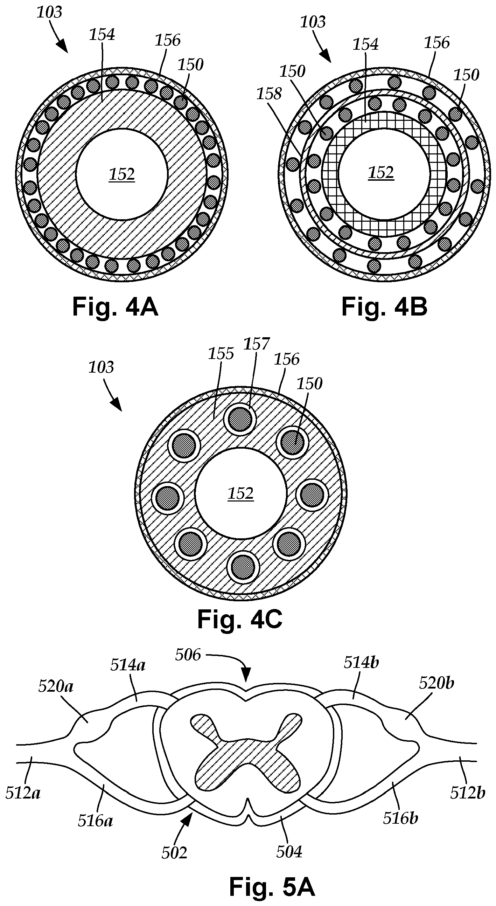

Figure 4A illustrates a cross-section of one embodiment of a lead 103 in which the conductors 150 are coiled together in a single layer. The lead 103 defines a central lumen 152 with a liner tube 154 between the central lumen and the conductors 150 and a jacket 156 disposed over the conductors.

Figure 4B illustrates a cross-section of another embodiment of a lead in which the conductors 150 are coiled together into two layers. The lead 103 defines a central lumen 152 and includes a liner tube 154 between the central lumen and the innermost layer of conductors 150, an optional liner 158 between the two layers of conductors 150, and a jacket disposed over the conductors. In at least some embodiments, the two layers of conductors 150 are wound in opposite direction (for example, the conductors in one layer are wound clockwise and the conductors in the other layer are wound counterclockwise). Such a counter-wound arrangement may result in higher torque transfer. In other embodiments, the two layers of conductors 150 may be wound in the same direction (e.g., either clockwise or counterclockwise). In at least some embodiments, the two layers of conductors 150 include the same number of conductors. In other embodiments, the two layers may include different numbers of conductors. The two layers of conductors 150 may be wound with the same or different pitch and at the same or different angle.

The conductors 150 can be made of any suitable conductive material and may be single wires or multi-filar cables or any other suitable conductive arrangement. The liner tube 154 can be made of any suitable polymer material including, but not limited to, ethylene tetrafluoroethylene (ETFE), polytetrafluoroethylene (PTFE), silicone, polyurethane, perfluoroalkoxy (PFA), or the like. The jacket 156 can be made of any suitable polymer material including, but not limited to, silicone, polyurethane, or the like. The optional liner 158 may be made of any suitable polymer material including, but not limited to, PFA, PTFE, EFTE, silicone, polyurethane, or the like.

Figure 4C illustrates a cross-section of yet another embodiment of a lead 103 that defines a central lumen 152 and includes a jacket 156, a multi-lumen conductor guide 155, and conductors 150 disposed in conductor lumens 157 formed in the multi-lumen conductor guide 155. In the illustrated embodiments, one conductor 150 is disposed in each conductor lumen, but in other embodiments, two, three, four or more conductors may be disposed in a conductor lumen.

In some embodiments, as an alternative to one or more of the conductors 150, an optical fiber or other optical waveguide for optical stimulation may extend along the lead. As an example, in an embodiment similar to Figure 4B, the conductors 150 are positioned in one of the layers and one or more optical fibers or other optical waveguides are positioned in the other layer. Alternatively, in an embodiment similar to Figure 4A, one or more optical fibers or other optical waveguides may extend through one or more lumens in the liner tube 154 or, in an embodiment similar to Figure 4C, in one or more of the conductor lumens 157.

A lead 103 may include any of the arrangements illustrated in Figures 4A to 4C and, in some embodiments, may include a combination of these arrangements with each arrangement in a different part of the lead. The arrangements of Figures 4A and 4B may be suitable for leads, or parts of a lead, with many conductors (for example, 10, 12, 16, 20, 30, 32, or more conductors). The arrangement of Figure 4C may be suitable for leads, or parts of a lead, with fewer conductors (for example, 2, 4, 6, 8, 10, 12, or 16 conductors).

Figure 5A schematically illustrates a transverse cross-sectional view of a spinal cord 502 surrounded by dura 504. The spinal cord 502 includes a midline 506 and a plurality of levels from which spinal nerves 512a and 512b extend. In at least some spinal cord levels, the spinal nerves 512a and 512b extend bilaterally from the midline 506 of the spinal cord 502. In Figure 5A, the spinal nerves 512a and 512b are shown attaching to the spinal cord 502 at a particular spinal cord level via corresponding dorsal roots 514a and 514b and corresponding ventral (or anterior) roots 516a and 516b. Typically, the dorsal roots 514a and 514b relay sensory information into the spinal cord 502 and the ventral roots 516a and 516b relay motor information outward from the spinal cord 502. The dorsal root ganglia (DRG) 520a and 520b are nodules of cell bodies that are disposed along the dorsal roots 516a and 516b in proximity to the spinal cord 502.

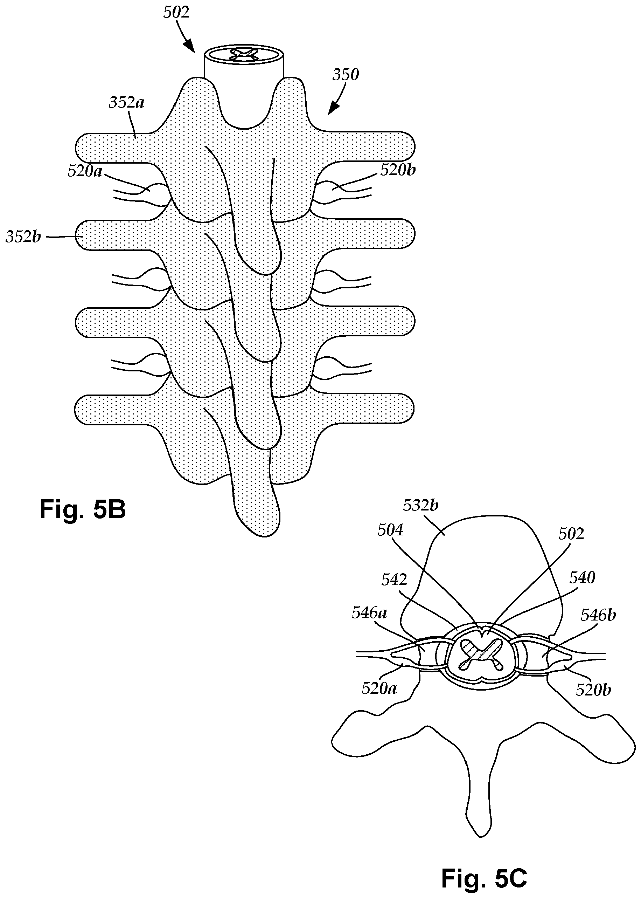

Figure 5B schematically illustrates a perspective view of a portion of the spinal cord 502 disposed along a portion of a vertebral column 530. The vertebral column 530 includes stacked vertebrae, such as vertebrae 532a and 532b, and a plurality of dorsal roots and DRGs 520a and 520b extending outwardly bilaterally from the spinal cord 502 at different spinal cord levels.

Figure 5C schematically illustrates a top view of a portion of the spinal cord 502 and surrounding dura 504 disposed in a vertebral foramen 540 defined in the vertebra 532b. The vertebrae, such as the vertebrae 532a and 532b, are stacked together and the vertebral foramina 540 of the vertebrae collectively form a spinal canal through which the spinal cord 502 extends. The space within the spinal canal between the dura 504 and the walls of the vertebral foramen 540 defines the epidural space 542. Intervertebral foramina 546a and 546b, defined bilaterally along sides of the vertebra 532b, form openings through the vertebra 532b between the epidural space 542 and the environment external to the vertebra 532b.

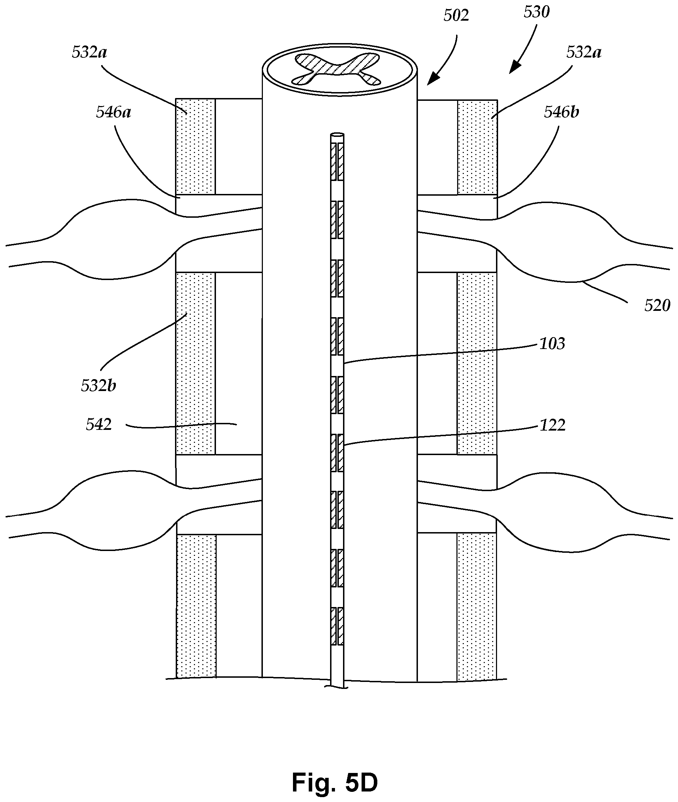

Figure 5D is a schematic perspective views of the spinal cord 502 disposed along a longitudinal transverse view of a portion of the vertebral column 530. The portion of the vertebral column 530 shown in Figure 5D includes the vertebrae 532a and 532b and intervertebral foramina 546a and 546b defined between the vertebrae 532a and 532b on opposing sides of the vertebral column 530. A DRG 520 extends outward from one side of the spinal cord 502 and through the intervertebral foramen 546b.

The lead 103 can be placed along the midline of the spinal column or elsewhere in the epidural space. In at least some embodiments, the lead 103 can be implanted through an introducer (not shown). Once the lead 103 is placed, the introducer can be removed or backed off. In the illustrated embodiment, the lead 103 includes multiple sets of two segmented electrodes 122. It will be recognized that a lead with any other combination of ring electrodes, segmented electrodes, tip electrode, or optical stimulator can be used including, but not limited to, the leads illustrated in Figures 3A to 3F.

Conventional ring electrodes stimulate all of the tissue surrounding the lead. This may be undesirable because the stimulation is not solely directed to the desired tissue to be stimulated and, therefore, results in a reduction in effective stimulation energy. Additionally or alternatively, in some instances, the stimulation may stimulate tissue that produces an undesirable side effect. The inclusion of segmented electrodes in a lead may facilitate directing stimulation to the desired tissue.

In Figure 5D, the lead 103, with one or more sets of two segmented electrodes, can be used to provide midline or lateral stimulation at a number of different positions along the spinal cord. Midline stimulation at a particular position can be obtained by using both electrodes of a particular set at the desired position. Lateral stimulation, either right or left, can be obtained by using one of the segmented electrodes (e.g., either right or left) at the desired position. In addition, it will be recognized that midline stimulation can be provided at one or more positions and lateral stimulation at one or more different positions. Thus, a clinician has increased flexibility in stimulation options and, in at least some instances, direct more of the stimulation to the desired tissue. In at least some embodiments, segmented electrodes can be used to produce both anodic and cathodic current using, for example, electrodes at the same longitudinal position (or adjacent or different longitudinal positions) which may, at least in some instances, provide stronger directionality. And, using multiple current sources, the amount of directionality even on a single lead can be incremental (e.g., start with one cathode at 100%, then add anodic current on adjacent segmented electrode in increments).

A lead with one or more sets of three segmented electrodes can also be used to provide midline or lateral stimulation at a number of different positions along the spinal cord. One of the segmented electrodes of each set can be positioned directly above the dorsal column and the other two segmented electrodes are then directed to the right and left of the midline position. Midline stimulation at a particular position can be obtained by using either a) the segmented electrode positioned directly above the dorsal column or b) all three electrodes of a particular set at the desired position. Lateral stimulation, either right or left, can be obtained by using one of the other two segmented electrodes (e.g., either right or left) at the desired position. In addition, it will be recognized that midline stimulation can be provided at one or more positions and lateral stimulation at one or more different positions. It will also be recognized that incremental anodic current may be provided in adjacent electrodes to fine tune the position of the stimulation in the medial-lateral direction.

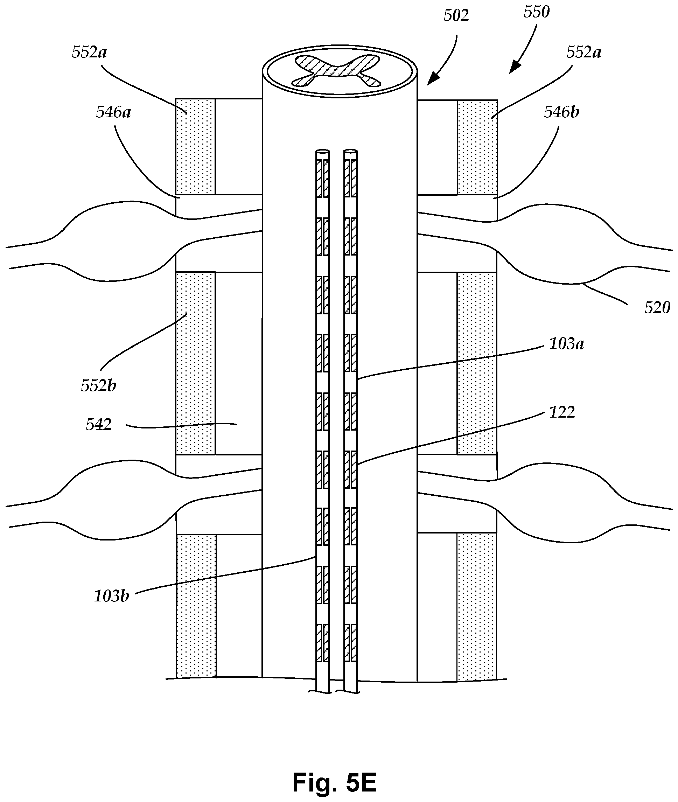

Conventional paddle leads can also provide midline or lateral stimulation, but these paddle leads typically use a much more invasive implantation procedure than percutaneous leads. The leads described herein can also be used to provide paddle-like stimulation in which multiple locations in a single row can be stimulated via electrodes at fixed relative distances but using leads that can be implanted percutaneously. As an example, in Figure 5E, two leads 103a, 103b, each with one or more sets of two or more segmented electrodes, can be used to provide paddle-like stimulation at a number of different positions along the spinal cord. In the illustrated embodiments, the leads 103a, 103b are implanted side-by-side to the same longitudinal depth along the spinal cord. It will be recognized that the leads 103a, 103b can be staggered so that one lead extends longitudinally further along the spinal cord than the other lead. It will also be recognized that the two leads 103a, 103b can have the same electrode configuration or can have different electrode configurations.

Midline and lateral stimulation at a particular position can be obtained by selecting electrodes of a particular set from one or both of the leads 103a, 103b at the desired position. Thus, midline, lateral, or any combination of midline and lateral stimulation can be provided using the two leads 103a, 103b. It will be recognized that other embodiments can include implantation of three or more leads.

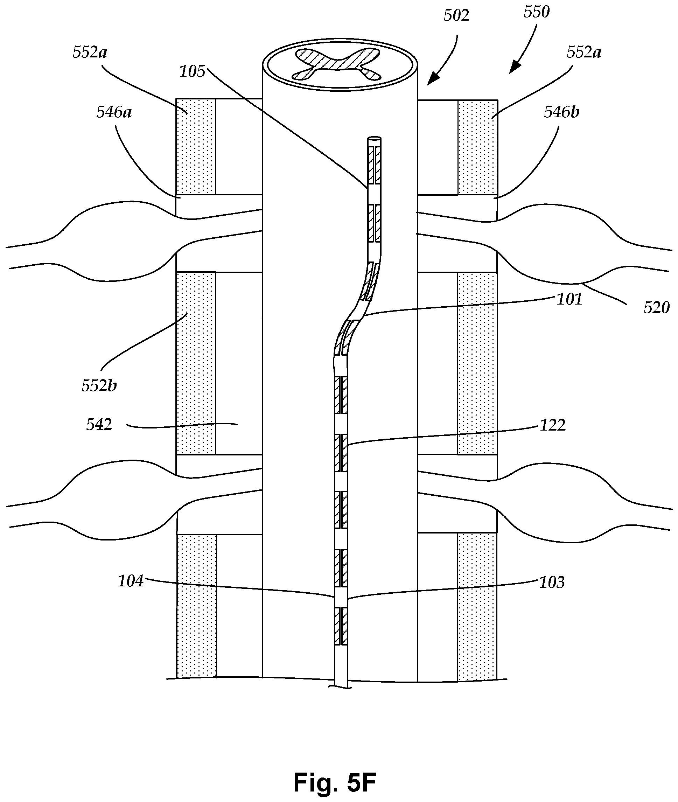

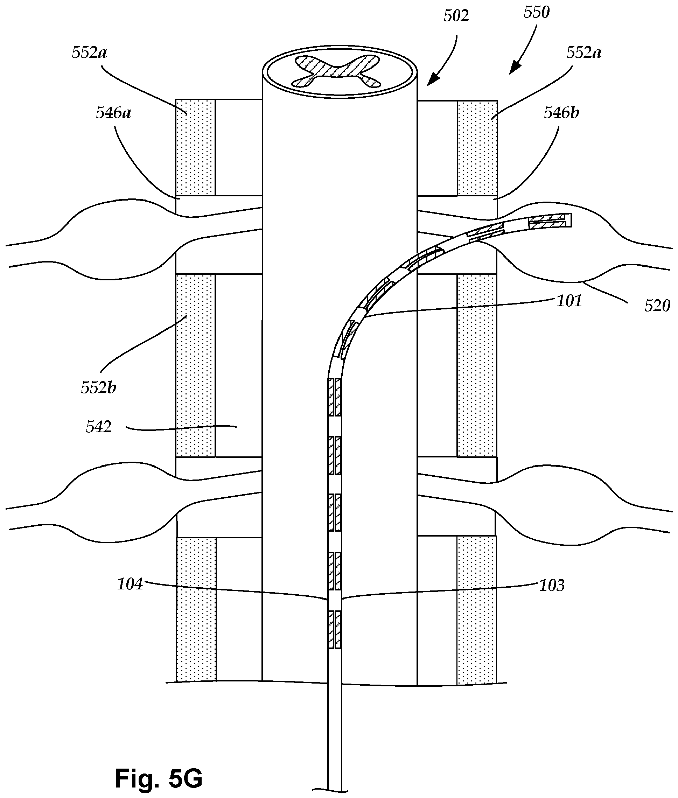

Figure 5F illustrates one embodiment of a portion of another lead 103 with a first distal straight region 104, a distal bent region 101, and an optional second distal straight region 105. Figure 5G illustrates another embodiment of a lead 103 with a distal straight region 104 and a distal bent region 101 (and may optionally include a second distal straight region (not shown)). These two lead embodiments are representative of leads that include electrodes positioned on both a distal straight region 104, 105 and a distal bent region 101. In at least some embodiments, multiple electrodes are disposed on each of the distal straight regions 104, 105, and the distal bent region 101, as illustrated in Figure 5F.

Leads 103, such as those illustrated in Figures 5F and 5G with a distal bent region, can be useful for providing stimulation to different structural elements of the spinal cord and adjacent structures. As illustrated in Figure 5F, the first distal straight region 104 of the lead 103 can be positioned on the midline of the spinal cord for stimulation the dorsal column. The distal bent region 101 of the lead 103 can then be used to position electrodes (on either the distal bent region or the second distal straight region 105 or both) over the dorsal horn, rootlet, or root for stimulation of that spinal cord structure. Figure 5F illustrates an arrangement with the lead 103 implanted so that the lead extends rostrally (e.g., upwards) along the vertebral column so that the second distal straight region 105 is further up along the vertebral column than the first distal straight region 104. In other embodiments, the lead 103 can be implanted retrograde so that the lead extends caudally (e.g., downwards) along the vertebral column so that the second distal straight region 105 is further down the vertebral column then the first distal straight region 104.

In Figure 5G, the first distal straight region 104 of the lead is disposed in the epidural space for stimulation of the spinal cord (for example, the dorsal column). The distal bent region 101 of the lead 103 exits the foramen 546b to position electrodes adjacent or near the dorsal root or dorsal root ganglion 520 for stimulation. In addition, electrodes on the distal bent region 101 within the epidural space may also be positioned for stimulating the dorsal horn, rootlet, or root.

In at least some embodiments, the leads 103 with a distal bent region 101 are permanently bent. In at least some embodiments, a stylet may be introduced during implantation to straighten the lead for part of the implantation procedure and then the stylet is removed to allow the lead to bend for its final position. In other embodiments, a bent stylet or other device may be used to form the distal bent region 101 in a lead 103. It will be understood that other leads may include multiple distal bent regions with the same or different degrees of bend.

A challenge with leads having more than eight electrodes is that conventional control modules have connectors with eight contacts. One option is a splitter for a sixteen-electrode lead that separates the proximal portion of the lead into two proximal ends with eight terminals on each end. The splitter may be part of the lead or of a lead extension to which the lead is attached. Another option is the use of segmented terminals, as described in, for example, U.S. Patents Nos. 9,656,093 and 9,833,611 and U.S. Patent Application Publication No. 2016/0228692, all of which are incorporated herein by reference in their entireties. Another option is to include more contacts in the connector of the control module, but this may increase the size of the control module.

The challenge becomes even greater for leads having more than sixteen electrodes. The electrode arrangement of the lead of Figure 3A with two ring electrodes and ten sets of three segmented electrodes (for a total of thirty-two electrodes) is used in the following for illustrative purposes, but it will be recognized that other lead configurations can be used.

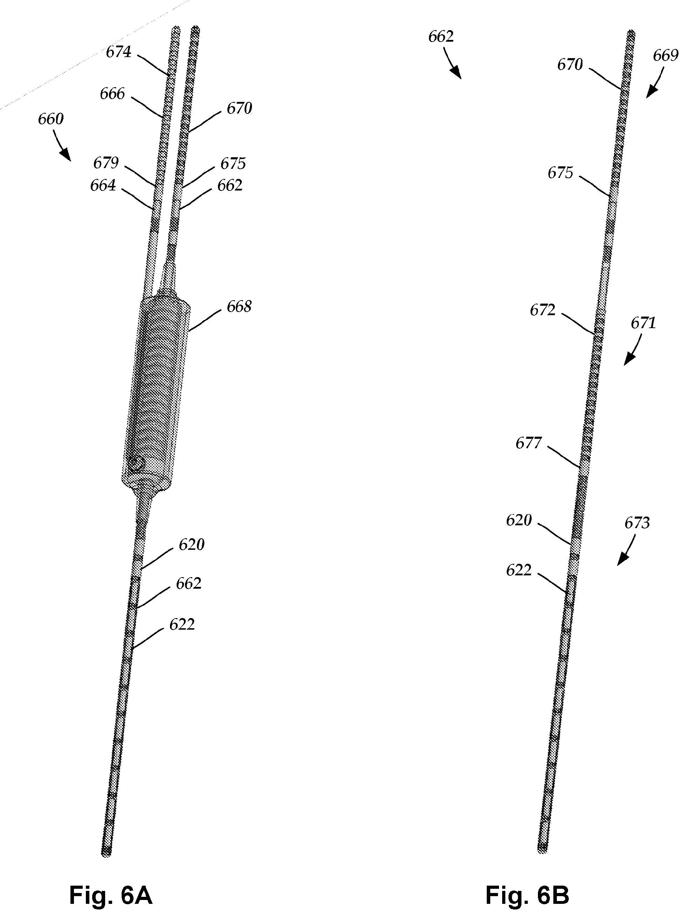

Figures 6A to 6D illustrate one embodiment of a lead arrangement 660 including a lead 662 and an extension 664 having a proximal portion 666 and a medial connector 668. Figure 6A illustrates the lead arrangement 660 when assembled.

Figure 6B illustrates the lead 662 which includes a proximal portion 669 with proximal terminals 670, a medial portion 671 with medial terminals 672, and a distal portion 673 with ring electrodes 620 and segmented electrodes 622. In at least some embodiments, the number of proximal terminals 670 equals the number of medial terminals 672. In other embodiments, the number of proximal terminals 670 can be larger or smaller than the number of medial terminals 672. In at least some embodiments, the total number of proximal terminals 670 and medial terminals 672 equals the number of electrodes 620, 622.

The lead 662 includes conductors 150 (Figures 4A to 4C) which couple to the ring electrodes 620 and segmented electrodes 622 to the proximal terminals 670 and medial terminals 672. In at least some embodiments, each electrode 620, 622 is electrically coupled by a conductor to either a proximal terminal 670 or a medial terminal 672. The lead may also include a proximal retention sleeve 675 and a medial retention sleeve 677 for coupling to a retention block in a connector of control unit/lead extension or the medial connector 668, respectively.

In at least some embodiments, the lead 662 includes a central lumen 152 (Figures 4A to 4C) that extends along a length of the lead and may be sealed at the distal end of the lead. In at least some embodiments, a portion of the lead 662 extending between at least the proximal terminals 670 and the medial terminals 672 may include a multi-lumen conductor guide 155 with conductor lumens 157 arranged around the central lumen 152, as illustrated in Figure 4C. Each conductor lumen may include one or more of the conductors that extend from the proximal terminals 670 to some of the electrodes 620, 622. Alternatively, the coiled conductor arrangements illustrated in Figures 4A or 4B can be used.

In at least some embodiments, a portion of the lead extending between at least the medial terminals 672 and the electrodes 620, 622 includes the conductors wound around the central lumen as illustrated in either Figure 4A or Figure 4B. In at least some embodiments that utilize the two layer arrangement illustrated in Figure 4B, the conductors coupled to the medial terminals 572 form one layer (for example, the outer layer) and the conductors coupled to the proximal terminals 570 form another layer (for example, the inner layer). Any other arrangement of the conductors may also be used.

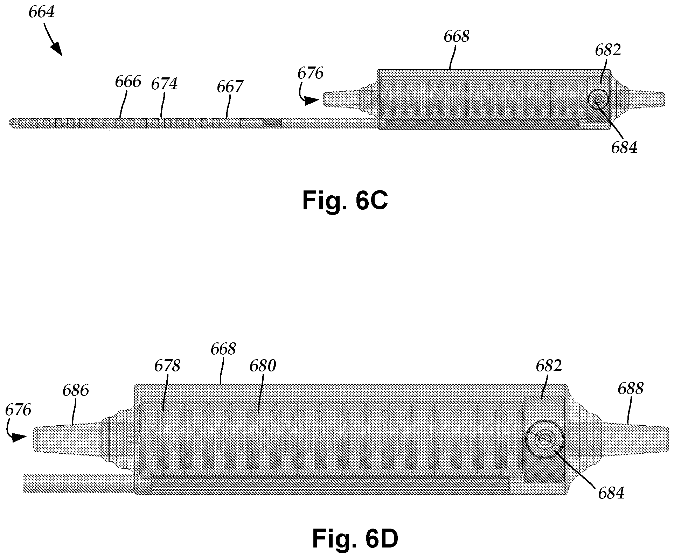

Figure 6C illustrates the extension 664 with the medial connector 668 and the proximal portion 666 having extension terminals 674 and an optional retention sleeve 679 disposed thereon. Figure 6D illustrates a closer view of the medial connector 668 which includes a central lumen 676 extending through the medial connector for receiving the lead 662, multiple connector contacts 678 which are arranged around the central lumen for electrically coupling to the medial terminals 672 of the lead, spacers 680 (or connector seals) disposed between the connector contacts, and an optional retention block 682 that includes a fastener 684 that can be fastened down on the medial retention sleeve 677 of the lead. The extension 664 includes conductor (not shown) that extend from the connector contact 678 to the extension terminals 674. The extension 664 may also include a proximal entrance element 686 and a distal entrance element 688 which can facilitate insertion of the lead 662 into the central lumen 676 of the medial connector 668 and may also provide strain relief.

In some embodiments, an inner diameter of a first portion of the central lumen 676 distal to the connector contacts 678 is larger than an inner diameter of a second portion of the central lumen proximal to the connector contacts to limit insertion of the lead 662 through the extension connector 668. Correspondingly, an outer diameter of the lead 662 distal to the medial terminals 672 may be larger than the outer diameter of the lead 662 in the region proximal to the medial terminals 672. Such an arrangement provides a stop for insertion of the lead 662 through the medial connector 668 because the larger diameter region of the lead cannot pass through the smaller diameter portion of the central lumen 676. Such an arrangement can facilitate the alignment of the medial terminals 672 of the lead 662 with the connector contacts 678 of the medial connector 668 of the extension 664.

For example, an inner diameter of the central lumen 676 may be larger at the distal entrance element 688 than for the proximal entrance element 686 and the portion of the medial connector 668 containing the connector contacts 678 and an outer diameter of the lead 662 may be larger distal to the medial terminals 672 than for the region containing, and proximal to, the medial terminals.

As another example, an inner diameter of the central lumen 676 may be larger at the distal entrance element 688 and through the portion of the medial connector 668 containing the connector contacts 678 than for the proximal entrance element 686 and an outer diameter of the lead 662 may be larger for the region containing, and distal to, the medial terminals 672 than for a region proximal to the medial terminals.

It will be recognized that the reverse arrangement can also be used with the inner diameter of the first portion of the central lumen 676 distal to the connector contacts 678 smaller than an inner diameter of the second portion of the central lumen proximal to the connector contacts and the outer diameter of the lead 662 distal to the medial terminals 672 smaller than the outer diameter of the lead 662 in the region proximal to the medial terminals 672.

In at least some embodiments, a transition from larger diameter to smaller diameter may provide a shoulder on the lead 662 and a corresponding shoulder in the medial connector 668. In at least some embodiments, the transition from larger to smaller diameter may occur at the proximal or distal end of the retention sleeve 677 or one of the medial terminals 672 or at any other suitable place along the lead 662.

In at least some embodiments, the proximal portion 666 of the extension 664 includes a central lumen 152 (Figures 4A to 4C). In at least some embodiments, a portion of the extension 664 extending between at least the extension terminals 672 and the medial connector 668 may include a multi-lumen conductor guide 155 with conductor lumens 157 arranged around the central lumen 152, as illustrated in Figure 4C. Each conductor lumen may include one or more of the conductors that extend from the extension terminals 674 to the connector contacts 678. Alternatively, the coiled conductor arrangements illustrated in Figures 4A or 4B can be used.

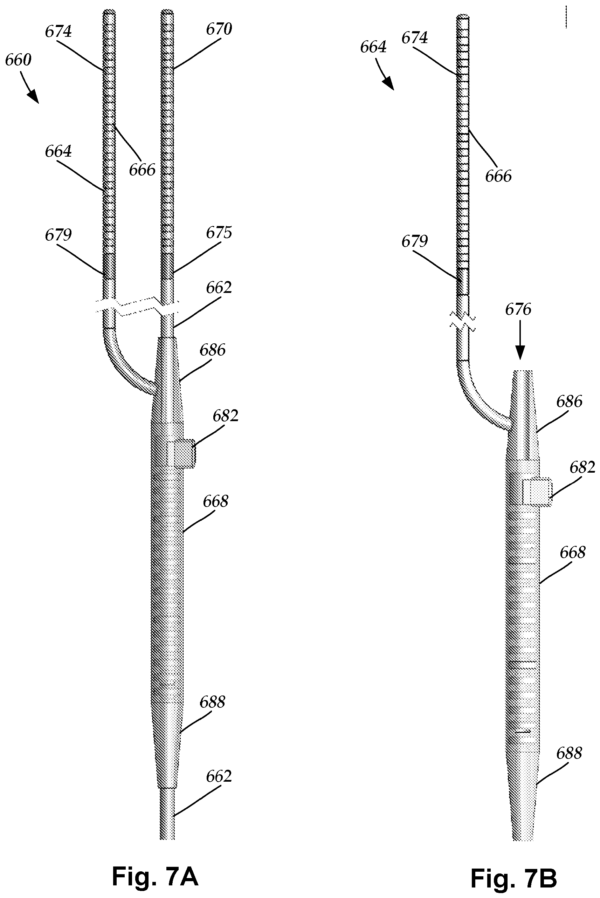

Figures 7A and 7B illustrate a portion of another embodiment of a lead arrangement 660 that is similar to the embodiment illustrated in Figures 6A to 6D with a variation in the coupling the proximal portion 666 of the extension 664 to the medial connector 668. In addition, as illustrated in Figures 7A and 7B, the retention block 682 of the medial connector 668 is proximal to the connector contacts 678 and, therefore, the retention sleeve 677 (Figure 6B) will be proximal to the medial terminals 672 (Figure 6B). It will be recognized that the position of the retention block 682 and retention sleeve 677 can be modified in either one of the embodiment of Figures 6A to 6D or the embodiment of Figures 7A and 7B to correspond to the arrangement illustrated for the other one of the embodiments. In some embodiments, a lead arrangement may include multiple extensions 664 that each attach to a different set of medial terminals 672 arranged along a lead 662. For example, an arrangement with a thirty-two electrode lead may include three extensions with eight proximal terminals each along with eight proximal terminals on the lead.

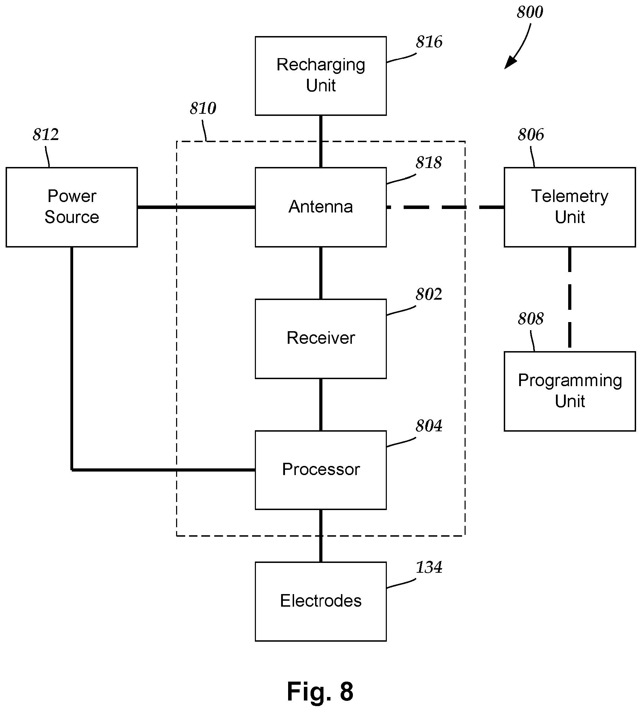

Figure 8 is a schematic overview of one embodiment of components of an electrical stimulation system 800 including an electronic subassembly 810 disposed within a control module. It will be understood that the electrical stimulation system can include more, fewer, or different components and can have a variety of different configurations including those configurations disclosed in the stimulator references cited herein.

Some of the components (for example, power source 812, antenna 818, receiver 802, and processor 804) of the electrical stimulation system can be positioned on one or more circuit boards or similar carriers within a sealed housing of an implantable pulse generator, if desired. Any power source 812 can be used including, for example, a battery such as a primary battery or a rechargeable battery. Examples of other power sources include super capacitors, nuclear or atomic batteries, mechanical resonators, infrared collectors, thermally-powered energy sources, flexural powered energy sources, bioenergy power sources, fuel cells, bioelectric cells, osmotic pressure pumps, and the like including the power sources described in U.S. Patent No. 8,437,193, incorporated herein by reference in its entirety.

As another alternative, power can be supplied by an external power source through inductive coupling via the optional antenna 818 or a secondary antenna. The external power source can be in a device that is mounted on the skin of the user or in a unit that is provided near the user on a permanent or periodic basis.

If the power source 812 is a rechargeable battery, the battery may be recharged using the optional antenna 818, if desired. Power can be provided to the battery for recharging by inductively coupling the battery through the antenna to a recharging unit 816 external to the user. Examples of such arrangements can be found in the references identified above.

In one embodiment, electrical current is emitted by the electrodes 134 on the lead body to stimulate nerve fibers, muscle fibers, or other body tissues near the electrical stimulation system. A processor 804 is generally included to control the timing and electrical characteristics of the electrical stimulation system. For example, the processor 804 can, if desired, control one or more of the timing, frequency, amplitude, width, and waveform of the pulses. In addition, the processor 804 can select which electrodes can be used to provide stimulation, if desired. In some embodiments, the processor 804 may select which electrode(s) are cathodes and which electrode(s) are anodes. In some embodiments, the processor 804 may be used to identify which electrodes provide the most useful stimulation of the desired tissue.

Any processor can be used and can be as simple as an electronic device that, for example, produces pulses at a regular interval or the processor can be capable of receiving and interpreting instructions from an external programming unit 808 that, for example, allows modification of pulse characteristics. In the illustrated embodiment, the processor 804 is coupled to a receiver 802 which, in turn, is coupled to the optional antenna 818. This allows the processor 804 to receive instructions from an external source to, for example, direct the pulse characteristics and the selection of electrodes, if desired.

In one embodiment, the antenna 818 is capable of receiving signals (e.g., RF signals) from an external telemetry unit 806 which is programmed by a programming unit 808. The programming unit 808 can be external to, or part of, the telemetry unit 806. The telemetry unit 806 can be a device that is worn on the skin of the user or can be carried by the user and can have a form similar to a pager, cellular phone, or remote control, if desired. As another alternative, the telemetry unit 806 may not be worn or carried by the user but may only be available at a home station or at a clinician's office. The programming unit 808 can be any unit that can provide information to the telemetry unit 806 for transmission to the electrical stimulation system 800. The programming unit 808 can be part of the telemetry unit 806 or can provide signals or information to the telemetry unit 806 via a wireless or wired connection. One example of a suitable programming unit is a computer operated by the user or clinician to send signals to the telemetry unit 806.

The signals sent to the processor 804 via the antenna 818 and receiver 802 can be used to modify or otherwise direct the operation of the electrical stimulation system. For example, the signals may be used to modify the pulses of the electrical stimulation system such as modifying one or more of pulse width, pulse frequency, pulse waveform, and pulse amplitude. The signals may also direct the electrical stimulation system 800 to cease operation, to start operation, to start charging the battery, or to stop charging the battery. In other embodiments, the stimulation system does not include an antenna 818 or receiver 802 and the processor 804 operates as programmed.

Optionally, the electrical stimulation system 800 may include a transmitter (not shown) coupled to the processor 804 and the antenna 818 for transmitting signals back to the telemetry unit 806 or another unit capable of receiving the signals. For example, the electrical stimulation system 800 may transmit signals indicating whether the electrical stimulation system 800 is operating properly or not or indicating when the battery needs to be charged or the level of charge remaining in the battery. The processor 804 may also be capable of transmitting information about the pulse characteristics so that a user or clinician can determine or verify the characteristics.

## Claims

1. A lead arrangement, comprising:
an electrical stimulation lead (662) having a proximal portion (669), a distal portion (673), and a medial portion (671) between the proximal portion and the distal portion, the electrical stimulation lead comprising
a plurality of electrodes (620, 622) disposed along the distal portion of the electrical stimulation lead,
a plurality of proximal terminals (670) disposed along the proximal portion of the electrical stimulation lead,
a plurality of medial terminals (672) disposed along the medial portion of the electrical stimulation lead,
a plurality of first conductors (150) extending along the electrical stimulation lead and electrically coupling some of the electrodes to the proximal terminals, and
a plurality of second conductors (150) extending along the electrical stimulation lead and electrically coupling some of the electrodes to the medial terminals,
wherein an outer diameter of a first portion of the electrical stimulation lead distal to the medial terminals and proximal to the electrodes is larger than an outer diameter of a second portion of the electrical stimulation lead proximal to the medial terminals; and
an extension (664) having a proximal portion (666) and a distal portion, the extension comprising
a plurality of extension terminals (674) disposed along the proximal portion of the extension, and
an extension connector (668) disposed along the distal portion of the extension, the extension connector comprising
a connector housing defining a central lumen (676) configured to receive the medial portion of the electrical stimulation lead, and
a plurality of connector contacts (678) disposed within the connector housing and along the central lumen,
wherein an inner diameter of a first portion of the central lumen distal to the connector contacts is larger than an inner diameter of a second portion of the central lumen proximal to the connector contacts to limit insertion of the electrical stimulation lead through the extension connector.

2. The lead arrangement of claim 1, wherein the extension connector further comprises a distal entrance element (688) disposed within the housing and defining a distal-most portion of the central lumen having the larger inner diameter.

3. The lead arrangement of any one of claims 1 or 2, wherein the extension connector further comprises a proximal entrance element (686) disposed within the housing and defining a proximal-most portion of the central lumen.

4. The lead arrangement of any one of claims 1 to 3, wherein the extension connector further comprises a plurality of spacers (680), each spacer disposed between adjacent ones of the connector contacts.

5. The lead arrangement of any one of claims 1 to 4, wherein the electrical stimulation lead comprises a multi-lumen conductor guide (155) disposed at least between the proximal terminals and the medial terminals, the multi-lumen conductor guide defining a plurality of conductor lumens (157) disposed around the central lumen with each of the conductor lumens comprising a portion of one or more of the second conductors disposed therein.

6. The lead arrangement of any one of claims 1 to 5, wherein the first conductors and at least a portion of the second conductors are disposed in a single layer, coiled arrangement between at least the medial terminals and the electrodes.

7. The lead arrangement of any one of claims 1 to 6, wherein the first conductors and at least a portion of the second conductors are disposed in a two layer, coiled arrangement between at least the medial terminals and the electrodes.

8. The lead arrangement of claim 7, wherein the two layer, coiled arrangement comprises a first layer and a second layer, wherein the first conductors are coiled in the first layer and the second conductors are coiled in the second layer.

9. The lead arrangement of any one of claims 1 to 8, wherein the plurality of electrodes comprises at least twenty electrodes.

10. The lead arrangement of any one of claims 1 to 9, wherein the electrical stimulation lead further comprises a proximal retention sleeve (675) disposed distal to the proximal terminals (670) and proximal to the medial terminals (672).

11. The lead arrangement of any one of claims 1 to 10, wherein the electrical stimulation lead further comprises a medial retention sleeve (677) disposed distal to the medial terminals (672) and proximal to the electrodes (620, 622).

12. The lead arrangement of claim 11, wherein the extension connector (668) comprises a retention block (682) disposed in the connector housing, the extension connector (668) including a fastener (684) configured for fastening the extension connector (668) to the medial retention sleeve (677) of the electrical stimulation lead

13. The lead arrangement of any one of claims 1 to 12, wherein the number of proximal terminals (670) is equal to the number of medial terminals (672).

14. The lead arrangement of any one of claims 1 to 13, wherein the combined number of proximal terminals (670) and medial terminals (672) equals the number of electrodes.

15. A system for electrical stimulation, the system comprising:
the lead arrangement of any one of claims 1 to 14;
a control module electrically coupleable to the electrical stimulation lead; and
optionally, further comprising a lead extension electrically coupleable between the electrical stimulation lead and the control module.

## Patentansprüche

1. Leitungsanordnung, mit:
einer elektrischen Stimulationsleitung (662) mit einem proximalen Abschnitt (669), einem distalen Abschnitt (673) und einem Mittenabschnitt (671) zwischen dem proximalen Abschnitt und dem distalen Abschnitt, wobei die elektrische Stimulationsleitung aufweist:
mehrere Elektroden (620, 622), die entlang des distalen Abschnitts der elektrischen Stimulationsleitung angeordnet sind;
mehrere proximale Anschlüsse (670), die entlang des proximalen Abschnitts der elektrischen Stimulationsleitung angeordnet sind;
mehreren Mittenanschlüsse (672), die entlang des Mittenabschnitts der elektrischen Stimulationsleitung angeordnet sind;
mehrere erste Leiter (150), die sich entlang der elektrischen Stimulationsleitung erstrecken und einige der Elektroden mit den proximalen Anschlüssen elektrisch verbinden; und
mehrere zweite Leiter (150), die sich entlang der elektrischen Stimulationsleitung erstrecken und einige der Elektroden mit den Mittenanschlüssen elektrisch verbinden,
wobei ein Außendurchmesser eines ersten Abschnitts der elektrischen Stimulationsleitung distal von den Mittenanschlüssen und proximal zu den Elektroden größer ist als ein Außendurchmesser eines zweiten Abschnitts der elektrischen Stimulationsleitung proximal zu den Mittenanschlüssen; und
einer Verlängerung (664) mit einem proximalen Abschnitt (666) und einem distalen Abschnitt, wobei die Verlängerung aufweist:
mehrere Verlängerungsanschlüsse (674), die entlang des proximalen Abschnitts der Verlängerung angeordnet sind; und
einen Verlängerungsverbinder (668), der entlang des distalen Abschnitts der Verlängerung angeordnet ist, wobei der Verlängerungsverbinder aufweist:
ein Verbindergehäuse, das ein mittiges Lumen (676) definiert, das dafür konfiguriert ist, den Mittenabschnitt der elektrischen Stimulationsleitung aufzunehmen; und
mehrere Verbinderkontakte (678), die innerhalb des Verbindergehäuses und entlang des mittigen Lumens angeordnet sind,
wobei ein Innendurchmesser eines ersten Abschnitts des mittigen Lumens distal von den Verbinderkontakten größer ist als ein Innendurchmesser eines zweiten Abschnitts des zentralen Lumens proximal zu den Verbinderkontakten, um ein Einführen der elektrischen Stimulationsleitung durch den Verlängerungsverbinder zu begrenzen.

2. Leitungsanordnung nach Anspruch 1, wobei der Verlängerungsverbinder ferner ein distales Eintrittselement (688) aufweist, das innerhalb des Gehäuses angeordnet ist und einen distalsten Abschnitt des mittigen Lumens mit dem größeren Innendurchmesser definiert.

3. Leitungsanordnung nach Anspruch 1 oder 2, wobei der Verlängerungsverbinder ferner ein proximales Eintrittselement (686) aufweist, das innerhalb des Gehäuses angeordnet ist und einen proximalsten Abschnitt des mittigen Lumens bildet.

4. Leitungsanordnung nach einem der Ansprüche 1 bis 3, wobei der Verlängerungsverbinder ferner mehrere Abstandselemente (680) aufweist, wobei jedes Abstandselement zwischen benachbarten der Verbinderkontakte angeordnet ist.

5. Leitungsanordnung nach einem der Ansprüche 1 bis 4, wobei die elektrische Stimulationsleitung eine mehrlumige Leiterführung (155) aufweist, die mindestens zwischen den proximalen Anschlüssen und den Mittenanschlüssen angeordnet ist, wobei die mehrlumige Leiterführung mehrere Leiterlumen (157) definiert, die um das mittige Lumen herum angeordnet sind, wobei jedes der Leiterlumen einen Abschnitt eines oder mehrerer der darin angeordneten zweiten Leiter aufweist.

6. Leitungsanordnung nach einem der Ansprüche 1 bis 5, wobei die ersten Leiter und mindestens ein Abschnitt der zweiten Leiter in einer einlagigen, gewickelten Anordnung zwischen mindestens den Mittenanschlüssen und den Elektroden angeordnet sind.

7. Leitungsanordnung nach einem der Ansprüche 1 bis 6, wobei die ersten Leiter und mindestens ein Abschnitt der zweiten Leiter in einer zweilagigen, gewickelten Anordnung zwischen mindestens den Mittenanschlüssen und den Elektroden angeordnet sind.

8. Leitungsanordnung nach Anspruch 7, wobei die zweilagige, gewickelte Anordnung eine erste Lage und eine zweite Lage aufweist, wobei die ersten Leiter in der ersten Lage und die zweiten Leiter in der zweiten Lage gewickelt sind.

9. Leitungsanordnung nach einem der Ansprüche 1 bis 8, wobei die mehreren Elektroden mindestens zwanzig Elektroden aufweisen.

10. Leitungsanordnung nach einem der Ansprüche 1 bis 9, wobei die elektrische Stimulationsleitung ferner eine proximale Haltehülse (675) aufweist, die distal von den proximalen Anschlüssen (670) und proximal zu den Mittenanschlüssen (672) angeordnet ist.

11. Leitungsanordnung nach einem der Ansprüche 1 bis 10, wobei die elektrische Stimulationsleitung ferner eine mittige Haltehülse (677) aufweist, die distal von den mittigen Anschlüssen (672) und proximal zu den Elektroden (620, 622) angeordnet ist.

12. Leitungsanordnung nach Anspruch 11, wobei der Verlängerungsverbinder (668) einen im Verbindergehäuse angeordneten Halteblock (682) aufweist, wobei der Verlängerungsverbinder (668) ein Befestigungselement (684) aufweist, das dafür konfiguriert ist, den Verlängerungsverbinder (668) an der mittigen Haltehülse (677) der elektrischen Stimulationsleitung zu befestigen.

13. Leitungsanordnung nach einem der Ansprüche 1 bis 12, wobei die Anzahl der proximalen Anschlüsse (670) der Anzahl der mittigen Anschlüsse (672) gleicht.

14. Leitungsanordnung nach einem der Ansprüche 1 bis 13, wobei die kombinierte Anzahl der proximalen Anschlüsse (670) und der mittigen Anschlüsse (672) der Anzahl der Elektroden gleicht.

15. System zur elektrischen Stimulation, wobei das System aufweist:
die Leitungsanordnung nach einem der Ansprüche 1 bis 14;
ein mit der elektrischen Stimulationsleitung elektrisch verbindbares Steuermodul; und
optional ferner eine Leitungsverlängerung, die zwischen der elektrischen Stimulationsleitung und dem Steuermodul elektrisch verbindbar ist.

## Revendications

1. Agencement de fils, comprenant :
un fil de stimulation électrique (662) ayant une partie proximale (669), une partie distale (673) et une partie médiale (671) entre la partie proximale et la partie distale, le fil de stimulation électrique comportant :
une pluralité d'électrodes (620, 622) disposées le long de la partie distale du fil de stimulation électrique ;
une pluralité de bornes proximales (670) disposées le long de la partie proximale du fil de stimulation électrique ;
une pluralité de bornes médiales (672) disposées le long de la partie médiale du fil de stimulation électrique ;
une pluralité de premiers conducteurs (150) s'étendant le long du fil de stimulation électrique et reliant électriquement certaines des électrodes aux bornes proximales ; et
une pluralité de deuxièmes conducteurs (150) s'étendant le long du fil de stimulation électrique et reliant électriquement certaines des électrodes aux bornes médiales ;
dans lequel un diamètre extérieur d'une première partie du fil de stimulation électrique, distale par rapport aux bornes médiales et proximale par rapport aux électrodes, est plus grand qu'un diamètre extérieur d'une deuxième partie du fil de stimulation électrique proximale par rapport aux bornes médiales ; et
une extension (664) ayant une partie proximale (666) et une partie distale, l'extension comprenant :
une pluralité de bornes d'extension (674) disposées le long de la partie proximale de l'extension ; et
un connecteur d'extension (668) disposé le long de la partie distale de l'extension, le connecteur d'extension comprenant :
un boîtier de connecteur définissant une lumière centrale (676), configuré de manière à recevoir la partie médiale du fil de stimulation électrique ; et
une pluralité de contacts de connecteur (678) disposés à l'intérieur du boîtier de connecteur et le long de la lumière centrale ;
dans lequel un diamètre intérieur d'une première partie de la lumière centrale, distale par rapport aux contacts de connecteur, est plus grand qu'un diamètre intérieur d'une deuxième partie de la lumière centrale proximale par rapport aux contacts de connecteur, afin de limiter l'insertion du fil de stimulation électrique à travers le connecteur d'extension.

2. Agencement de fils selon la revendication 1, dans lequel le connecteur d'extension comprend en outre un élément d'entrée distale (688) disposé à l'intérieur du boîtier et définissant une partie la plus distale de la lumière centrale ayant le plus grand diamètre intérieur.

3. Agencement de fils selon l'une quelconque des revendications 1 et 2, dans lequel le connecteur d'extension comprend en outre un élément d'entrée proximale (686) disposé à l'intérieur du boîtier et définissant une partie la plus proximale de la lumière centrale.

4. Agencement de fils selon l'une quelconque des revendications 1 à 3, dans lequel le connecteur d'extension comprend en outre une pluralité d'entretoises (680), chaque entretoise étant disposée entre des contacts de connecteur adjacents parmi les contacts de connecteur.

5. Agencement de fils selon l'une quelconque des revendications 1 à 4, dans lequel le fil de stimulation électrique comprend un guide conducteur multi-lumière (155) disposé au moins entre les bornes proximales et les bornes médiales, le guide conducteur multi-lumière définissant une pluralité de lumières conductrices (157) disposées autour de la lumière centrale, où chacune des lumières conductrices comprend une partie d'un ou plusieurs des deuxièmes conducteurs disposés à l'intérieur.

6. Agencement de fils selon l'une quelconque des revendications 1 à 5, dans lequel les premiers conducteurs et au moins une partie des deuxièmes conducteurs sont disposés dans un agencement enroulé à une seule couche entre au moins les bornes médiales et les électrodes.

7. Agencement de fils selon l'une quelconque des revendications 1 à 6, dans lequel les premiers conducteurs et au moins une partie des deuxièmes conducteurs sont disposés dans un agencement enroulé à deux couches entre au moins les bornes médiales et les électrodes.

8. Agencement de fils selon la revendication 7, dans lequel l'agencement enroulé à deux couches comprend une première couche et une deuxième couche, dans lequel les premiers conducteurs sont enroulés dans la première couche et les deuxièmes conducteurs sont enroulés dans la deuxième couche.

9. Agencement de fils selon l'une quelconque des revendications 1 à 8, dans lequel la pluralité d'électrodes comprend au moins vingt électrodes.

10. Agencement de fils selon l'une quelconque des revendications 1 à 9, dans lequel le fil de stimulation électrique comprend en outre un manchon de retenue proximale (675) disposé de manière distale par rapport aux bornes proximales (670) et de manière proximale par rapport aux bornes médiales (672).

11. Agencement de fils selon l'une quelconque des revendications 1 à 10, dans lequel le fil de stimulation électrique comprend en outre un manchon de retenue médiale (677) disposé de manière distale par rapport aux bornes médiales (672) et de manière proximale par rapport aux électrodes (620, 622).

12. Agencement de fils selon la revendication 11, dans lequel le connecteur d'extension (668) comprend un bloc de rétention (682) disposé dans le boîtier de connecteur, le connecteur d'extension (668) incluant une attache (684) configurée de manière à attacher le connecteur d'extension (668) au manchon de retenue médiale (677) du fil de stimulation électrique.

13. Agencement de fils selon l'une quelconque des revendications 1 à 12, dans lequel le nombre de bornes proximales (670) est égal au nombre de bornes médiales (672).

14. Agencement de fils selon l'une quelconque des revendications 1 à 13, dans lequel le nombre combiné de bornes proximales (670) et de bornes médiales (672) est égal au nombre d'électrodes.

15. Système de stimulation électrique, le système comprenant :
l'agencement de fils selon l'une quelconque des revendications 1 à 14 ;
un module de commande pouvant être couplé électriquement au fil de stimulation électrique ; et
facultativement, comprenant en outre une extension de fil pouvant être couplée électriquement entre le fil de stimulation électrique et le module de commande.
